# EUROPEAN PATENT APPLICATION

(11) **EP 0 534 019 A1**
(43) Date of publication of application: **31.03.1993**
(21) Application number: 91308648.4
(22) Date of filing: 23.09.1991
(51) Int. Cl.: C07C 37/00

(54) **Isomerization of alkylphenols**

(71) Applicant: UOP, Des Plaines, Illinois 60017-5017 (US)
(72) Inventor: Sachtler, J. W. Adriaan, Des Plaines, Illinois 60018 (US); Lawson, Joe R., Palatine, Illinois 60067 (US)
(74) Representative: Brock, Peter William

(57) **Abstract**

A nonequilibrium mixture of alkylphenols is isomerized by contacting hydrogen and the mixture at isomerization conditions with a catalyst comprising a combination of a platinum group metal and a modifier selected from lead, tin, germanium or bismuth with a carrier containing a pentasil zeolite and an inorganic oxide binder.

## Description

### FIELD OF THE INVENTION

This invention relates to a process for isomerizing a nonequilibrium mixture of alkylphenols such as cresols using a novel catalyst system containing a platinum group metal, a modifier selected from lead, tin, germanium or bismuth and a pentasil zeolite such as ZSM-5.

### BACKGROUND OF THE INVENTION

The individual alkylphenol isomers have a wide variety of uses. Para-cresol, or p-cresol, is particularly useful in disinfectants or fumigating compositions and in the synthesis of such compounds as cresotimic acid and dyestuffs. Ortho-cresol, or o-cresol, finds application as a disinfectant or in the preparation of coumarine and other organic intermediates. Meta-cresol, or m-cresol, is used in photographic and ore-flotation chemicals, paint and varnish removers, disinfectant and fumigating compositions and in the production of synthetic resins and explosives.

The relative proportions of cresol isomers in a natural or synthetic mixture of cresols would not be expected to match the proportions of each cresol isomer needed to meet product requirements. This divergence between the supply and demand proportions of cresol isomers is the driving force favoring development of a cresol isomerization process to convert surplus isomers to needed isomers.

Bound zeolite catalysts are widely used for conversion reactions, based on their high activity and/or selectivity. Zeolites, or crystalline aluminosilicates, may be represented by the empirical formula:

M_{2/n}O · Al₂O₃ · xSiO₂ · yH₂O

in which n is the valence of M and x is generally equal to or greater than 2. Zeolites have skeletal structures which are made up of three-dimensional networks of SiO₄ and AlO₄ tetrahedra, corner linked to each other by shared oxygen atoms. Zeolites particularly suited for use as isomerization catalysts include mordenite and the ZSM variety.

Isomerization of o-cresol using a zeolite of the ZSM-type is disclosed in U.S. Patent 4,283,571 and U.S. Patent 4,503,269. When the prior art is compared with the subject matter of the present invention, it is believed that the present process for isomerizing alkylphenols is neither taught nor suggested. The unique combination of Group VIII metal, such as a platinum group metal, a modifier such as lead, and relatively low pentasil zeolite content on an inorganic binder exhibits surprising results in isomerizing cresols with high selectivity.

### DESCRIPTION OF THE INVENTION

### Objects

It is an object of the present invention to provide a novel process for the isomerization of alkylphenols. A corollary objective of the invention is to provide an isomerization process which isomerizes cresols at high yield efficiency.

### Summary of the Invention

This invention is based on the discovery that a bound zeolite catalyst containing a platinum group metal and a modifier selected from the group consisting of lead, tin, germanium and bismuth demonstrates unexpectedly high yield efficiency when isomerizing a mixture of alkylphenols such as cresols to increase the yield of desired isomers.

### Embodiments

A broad embodiment of the present invention is an alkylphenol isomerization process wherein a nonequilibrium mixture of alkylphenols and hydrogen are contacted at isomerization conditions with a catalyst comprising a combination of a platinum group metal and a modifier selected from the group consisting of lead, tin, germanium or bismuth with a carrier material containing a pentasil zeolite and an inorganic binder.

In a preferred embodiment, the feedstock to the isomerization process comprises a non-equilibrium mixture of cresol isomers.

In a highly preferred embodiment, the alkylphenol isomerization process utilizes a catalyst comprising a combination of a platinum component and a lead component with a carrier material containing from 1 to 20 mass % of a ZSM-5 zeolite and an alumina binder wherein the atomic ratio of lead to platinum is from 2:1 to about 10:1.

These as well as other embodiments will become apparent upon a reading of the following detailed description of the invention.

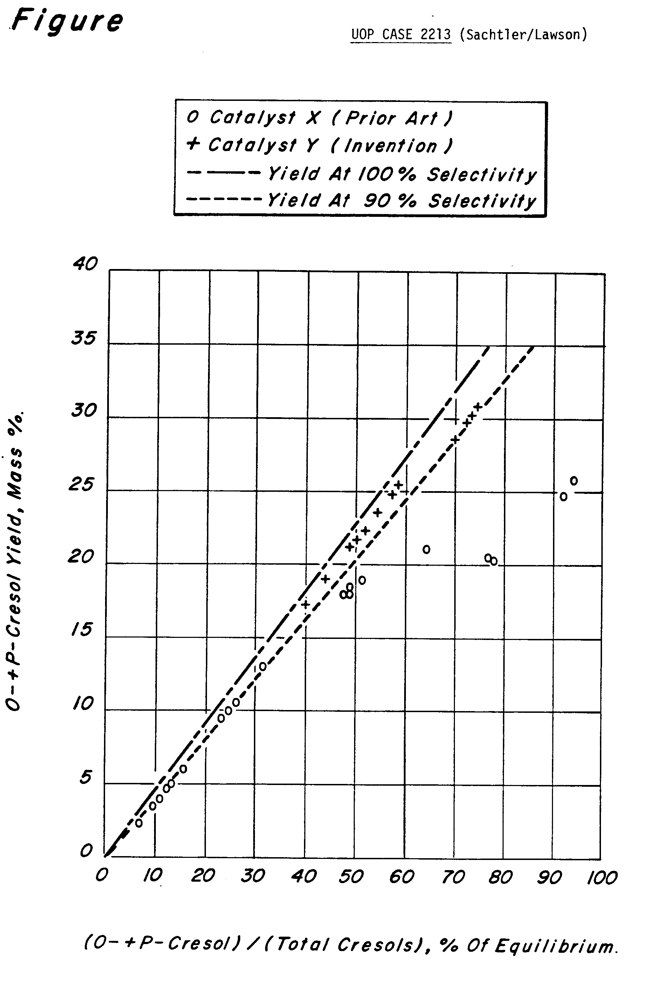

### BRIEF DESCRIPTION OF THE DRAWING

The accompanying drawing shows the surprising benefit of the subject invention. The "Figure" shows the yield efficiency of desired cresol isomers in relation to conversion or extent of isomerization.

### DETAILED DESCRIPTION OF THE INVENTION

The present invention is an alkylphenol isomerization process using a catalyst comprising a metal component from Group VIII of the Periodic Table (see Cotton and Wilkinson, Advanced Inorganic Chemistry, (3rd ed., 1972)) and a modifier on a carrier material comprising a pentasil zeolite and an inorganicoxide binder.

The process of this invention finds utility in the isomerization of isomerizable alkylphenols. Alkylphenols are phenols with one or more hydrocarbon sidechains attached to the aromatic ring. The hydrocarbon sidechains may be CH₃, C₂H₅, C₃H₇, or C₄H₉ or have five or more carbon atoms. The alkylphenols thus may be, for example, cresols, xylenols, phlorol and other ethylphenols, pseudocumenol, mesitol, methylethylphenols, propylphenols, and higher alkylphenols. The feedstock to the present process may contain more than one species of alkylphenol and may contain other constituents which do not substantially interfere with the isomerization reaction. Cresols comprise the preferred feedstock to the present process.

The isomerizable alkylphenols, preferably comprising cresols, may be utilized as found in selective fractions from various refinery petroleum streams or streams derived from coal or wood processing or from upgrading of synthesis gas. For example, the feedstock to the present process may be recovered from cracking of heavy petroleum fractions, pyrolysis of petroleum distillates, caustic extraction of petroleum fractions, extraction of coal tar, or separation of Fischer-Tropsch products. The feedsrock also may be derived by alkylation of phenol, e.g., with an olefin or alcohol. The preferred cresol-containing feedstock comprises one or more of the isomers p-cresol, o-cresol and m-cresol.

According to the process of the present invention, an alkylphenol charge stock, preferably in admixture with hydrogen, is contacted with a catalyst of the type described hereinafter at isomerization conditions. Contacting may be effected using the catalyst in a fixed-bed system, a moving-bed system, a fluidized-bed system, or in a batch-type operation. A batch-type operation is described in U.S. Patent 4,503,269, incorporated herein by reference thereto. In view of the danger of attrition loss of the valuable catalyst and of operational advantages, it is preferred to use a fixed-bed system. In this system, a hydrogen-rich gas and the feedstock are preheated by suitable heating means to the desired reaction temperature and then passed into an isomerization zone containing a fixed bed of the hereinafter-characterized catalyst. The conversion zone may be one or more separate reactors with suitable means therebetween to ensure that the desired isomerization temperature is maintained at the entrance to each zone. It is to be noted that the reactants may be contacted with the catalyst bed in either upward, downward, or radial-flow fashion, and that the reactants may be in the liquid phase, a mixed liquid-vapor phase, or a vapor phase when contacted with the catalyst.

The subject process is preferably effected by contacting the alkylphenol-containing feedstock at isomerization conditions with a fixed bed of the hereinafter-described catalyst by passing the hydrocarbon in a down-flow fashion through the bed. Isomerization conditions include a temperature in the range of from about 0° to 600°C and a pressure of from 101.3 to 10130 kPa (1 to 100 atmospheres). The temperature range preferably is 250 to 500°C, especially 300°-450°C, and a pressure range of 101.3 to 7598 kPa (1-75 atmospheres) is employed. The hydrocarbon is passed into the reaction zone preferably in admixture with hydrogen at a hydrogen to alkylphenol mole ratio of 0.5:1 to 15:1 or more. Other inert diluents such as nitrogen, argon, methane, ethane, and the like may be present. The liquid hourly hydrocarbon space velocity of the feedstock relative to the volume of catalyst is from 0.5 to 30 hr⁻¹, and most preferably at 1 to 20 hr⁻¹.

The particular product recovery scheme employed is not deemed to be critical to the instant invention. Any recovery scheme known in the art may be used. Typically, the reactor effluent will be condensed with the hydrogen and other light components removed therefrom by flash separation. The condensed liquid product is then subjected to a fractionation procedure to further purify the desired liquid isomerized product. Isomerized product derived from the processing of the preferred cresol-containing feedstock preferably is further fractionated to separate one or more substantially pure cresol isomers, i.e., p-cresol, o-cresol or m-cresol. Alternatively, the cresol isomers may be separated by crystallization methods or most preferably by selective adsorption using crystalline aluminosilicates. Adsorptive processes for separating cresols are described, for example, in U.S. Patents 3,014,078; 3,969,422 and 4,356,331, incorporated herein by reference thereto. The substantially pure cresol isomers are suitable for formulations and chemical syntheses such as those mentioned hereinabove. The raffinate remaining after recovery of the desired cresol isomers may be returned to the isomerization reactor section.

As mentioned, the catalyst of the present invention contains a pentasil zeolite. "Pentasil" is a term used to describe a class of shape-selective zeolites. This novel class of zeolites is well known to the art and is typically characterized by a silica/alumina mole ratio of at least about 12. Descriptions of the pentasils may be found in U.S. Patents 4,159,282; 4,163,018; and 4,278,565, all of which are incorporated herein by reference. Of the pentasil zeolites, the preferred ones are ZSM-5, ZSM-8, ZSM-11, ZSM-12, ZSM-23, and ZSM-35, with ZSM-5 being particularly preferred. It is a preferred embodiment of the present invention that the pentasil be in the hydrogen form. Conversion of an alkali metal form pentasil to the hydrogen form may be performed by treatment with an aqueous solution of a mineral acid. Alternatively, hydrogen ions can be incorporated into the pentasil by ion exchange with ammonium cations, followed by calcination.

The relative proportion of pentasil zeolite in the carrier material of the catalyst composite is an important feature of the present invention. The pentasil zeolite content of the carrier material preferably ranges from 1 to 20 mass %, with 5 to 15 mass % preferred. There is a tradeoff between the zeolite content of the catalyst composite and the pressure and temperature of an isomerization operation in maintaining low cresol losses. Higher pressure requires higher temperature and lower zeolite content in order to avoid saturation and subsequent hydrocracking of aromatic compounds. The balance of the three parameters may result in a different optimum zeolite content for an isomerization unit designed after the present invention than for an existing unit with fixed pressure and temperature limitations.

It is also within the scope of the present invention that the particular pentasil selected may be a gallosilicate. Gallosilicates have essentially the same structure as the ZSM-type zeolites described hereinabove, except that all or part of the aluminum atoms in the aluminosilicate crystal framework are replaced by gallium atoms. This substitution of the aluminum by gallium is usually performed prior to or during synthesis of the zeolite. The gallium content for this particular type of pentasil, expressed as mole ratios of SiO₂ to Ga₂O₃, ranges from 20:1 to 400:1 or more.

The inorganic oxide binder utilized in the carrier material is a porous, adsorptive, high-surface area support having a surface area of about 25 to about 500 m²/g. The binder should also be uniform in composition and relatively refractory to the conditions utilized in the conversion process. By the term "uniform in composition", it is meant that the support be unlayered, has no concentration gradients of the species inherent to its composition, and is completely homogeneous in composition. Thus, if the support is a mixture of two or more refractory materials, the relative amounts of these materials will be constant and uniform throughout the entire support. It is intended to include within the scope of the present invention binder materials which have traditionally been utilized in dual-functional hydrocarbon conversion catalysts such as: (1) activated carbon, coke, or charcoal; (2) silica or silica gel, silicon carbide, clays and silicates including those synthetically prepared and naturally occurring, which may or may not be acid treated, for example, attapulgus clay, diatomaceous earth, fuller's earth, kaolin, kieselguhr, etc.; (3) ceramics, porcelain, bauxite; (4) refractory inorganic oxides such as alumina, titanium dioxide, zirconium dioxide, chromium oxide, zinc oxide, magnesia, thoria, boria, silica-alumina, silica-magnesia, chromia-alumina, alumina-boria, silica-zirconia, zirconia-alumina, etc.; and (5) combinations of one or more elements from one or more of these groups. The preferred binders for use in the present invention are refractory inorganic oxides, with best results obtained with a binder comprised of alumina. Suitable aluminas are the crystalline aluminas known as the gamma-, eta-, and theta-aluminas, with gamma-alumina as the preferred form. In addition, in some embodiments, the alumina binder may contain minor proportions of other well known refractory inorganic oxides such as silica, zirconia, magnesia, etc.; however, the preferred binder is substantially pure gamma-alumina. Preferred binders have an apparent bulk density of 0.3 to 0.8 g/cc and surface area characteristics such that the average pore diameter is 20 to 300 angstroms and the pore volume is 0.1 to 1 cc/g. In general, excellent results are typically obtained when the binder of the catalyst is gamma-alumina in the form of spherical or extruded particles having a relatively small diameter (i.e., typically 1/16-inch), an apparent bulk density of 0.4-0.7 g/cc, a pore volume of 0.7 cc/g, and a surface area of 200-270 m²/g.

The preferred alumina binder is uniform in composition and may be prepared in any suitable manner and may be synthetically prepared or naturally occurring. Whatever type of alumina is employed, it may be activated prior to use by one or more treatments including drying, calcination, steaming, etc., and it may be in a form known as activated alumina, activated alumina of commerce, porous alumina, alumina gel, etc. For example, the alumina binder may be prepared by adding a suitable alkaline reagent, such as ammonium hydroxide to a salt of aluminum such as aluminum chloride, aluminum nitrate, etc., in an amount to form an aluminum hydroxide gel which, upon drying and calcining, is converted to alumina.

Using techniques commonly known to those skilled in the art, the catalyst of the present invention may be composited and shaped into any useful form such as spheres, pills, cakes, extrudates, powders, granules, tablets, etc., and utilized in any desired size. These shapes may be prepared utilizing any known forming operations including spray drying, tabletting, spherizing, extrusion, and nodulizing. A preferred shape for the catalyst composite is the extrudate prepared using the well-known extrusion method. Here the pentasil zeolite is combined with the binder and a suitable peptizing agent and mixed to form a homogeneous dough or thick paste. The modifier may be added to the binder before compositing or to the mixture before shaping, either before, after, or simultaneously with the pentasil zeolite, in one embodiment as discussed later in more detail. This material is then extruded through a die pierced with multiple holes and the spaghetti-shaped extrudate is cut off on the opposite side to form short cylinders. The rheological properties of the dough mixture can be varied by the use of "extrusion aids" such as methylcellulose, stearates, small amounts of clay, colloidal silica, etc. After extrusion, the cylinders are dried and calcined as set forth hereinbelow.

An alternative preferred shape of the subject catalytic composite is the sphere, manufactured by the well-known oil-drop method which comprises forming a hydrosol of the desired inorganic oxide binder by any of the techniques taught in the art. For example, alumina hydrosol is preferably prepared by reacting aluminum metal with hydrochloric acid. The pentasil zeolite is then uniformly dispersed in the hydrosol. This resultant zeolite-containing hydrosol is then commingled with a suitable gelling agent and is dispersed as droplets into an oil bath maintained at elevated temperatures. As discussed later, in one embodiment, the modifier may be added to the mixture prior to forming the droplets and either before, after, or simultaneously with the pentasil. The droplets of the mixture remain in the oil bath until they set and form hydrogel spheres. The spheres are continuously withdrawn from the oil bath and typically subjected to specific aging treatments in oil and an ammoniacal solution to further improve their physical characteristics. The resulting aged and gelled particles are then washed and dried at a relatively low temperature of about 100-205°C and subjected to a calcination procedure at a temperature of about 450-700°C for a period of about 1 to about 20 hours. See the teachings of U.S. Patent 2,620,314 for additional details.

Another component of the present invention is the platinum group metal component. Preferably this platinum group metal is selected from platinum, palladium, rhodium, ruthenium, osmium, iridium and mixtures thereof. The preferred platinum-group metal is platinum, with palladium being the next preferred metal. The platinum-group metal component may exist within the final catalyst composite as a compound such as an oxide, sulfide, halide, oxysulfide, etc., or as an elemental metal or in combination with one or more other ingredients of the catalyst. It is believed that the best results are obtained when substantially all the platinum-group metal exists in a reduced state. The platinum-group metal generally comprises from 0.01 to 2 mass % of the final catalytic composite, calculated on an elemental basis. It is preferred that the platinum content of the catalyst be between 0.05 and 1 mass %.

The platinum-group metal component may be incorporated into the catalyst composite in any suitable manner, such as by ion-exchange or impregnation of the zeolite/binder composite. The preferred method of preparing the catalyst normally involves the utilization of a water-soluble, decomposable compound of a platinum-group metal to impregnate the calcined zeolite/binder composite. For example, the platinum-group metal component may be added to the calcined hydrogel by commingling the calcined composite with an aqueous solution of chloroplatinic or chloropalladic acid.

Another essential constituent of the present invention is a modifier. The modifier may be incorporated into the catalytic composite in any suitable manner to effectively disperse this component. Suitable methods could include coprecipitation or cogelation with the inorganic oxide binder with or without the zeolite, ion-exchange with the inorganic oxide binder, or impregnation of the catalyst at any stage in the preparation. The modifier may be selected from the group consisting of lead, tin, germanium and bismuth. The preferred modifier of the catalyst is a lead component.

One effective method of incorporating the preferred lead component into the catalytic composite involves the addition of suitable soluble lead compounds such as lead nitrate, lead acetate, lead citrate, lead formate, and the like to the zeolite-containing hydrosol of the inorganic oxide, and then combining the hydrosol with a suitable gelling agent and dispersing the resulting mixture into an oil bath with subsequent processing as explained in more detail hereinabove. After calcining the gelled hydrosol, a binder material is obtained having a uniform dispersion of lead oxide in an intimate combination principally with the inorganic oxide binder. Another preferred method of incorporating the lead component into the catalyst composite involves the utilization of a soluble, decomposable compound of lead to impregnate and uniformly disperse the lead on the composite. Best results are ordinarily obtained with a solution of lead nitrate and nitric acid. In general, the lead component can be impregnated either prior to, simultaneously with, or after the platinum-group metallic component is added to the carrier material. A preferred method is to impregnate the lead component simultaneously with the platinum-group metal component. A preferred impregnation solution contains chloroplatinic acid, nitric acid, and lead nitrate.

Regardless of which lead compound is used in the preferred impregnation step, it is important that the lead component be uniformly distributed throughout the carrier material. That is, it is important that the concentration of lead in any reasonably divisible portion of the carrier material be approximately the same. In order to achieve this objective, it is necessary to maintain the pH of the impregnation solution in a range of from 7 to about 1 or less. Good platinum-lead interaction results when the nitric acid content of the impregnated carrier material is from about 1 to about 15 mass %, and a nitric acid content from about 3 to about 11 mass % is preferred.

The effective dispersion of the platinum group metal and modifier components is essential to obtain the selectivity demonstrated by the catalyst of the present invention. It is believed, without limiting the present invention, that effective dispersion of the metals and avoidance of platinum crystallites results in association of the platinum metal and modifier with resulting beneficial attenuation of the activity of the platinum group metal. Such attenuation is believed to enhance catalyst selectivity by reducing losses to byproducts. Optimum interaction between platinum group metal and modifier components has been estimated for a large number of catalyst formulations and preparation techniques using a microreactor test of the conversion of methylcyclohexane to toluene at 450°C and 1 atm. pressure, with 1-40% conversion, and preferably 10-30% conversion being a target value.

The amount of the modifier component is optimally established as a function of the amount of platinum group metal contained in the catalyst composite. More specifically, unanticipated beneficial interaction of the platinum group metal component and modifier component is effected at an atomic ratio of modifier to platinum group metal of from about 2:1 to 10:1.

An optional constituent of the bimetallic catalyst used in the present invention is the halogen component. Although the precise form of the chemistry of the association of the halogen component with the carrier material is not entirely known, it is customary in the art to refer to the halogen component as being combined with the carrier material or with the other ingredients of the catalyst in the form of the corresponding halide (e.g. as the chloride or the fluoride). This combined halogen may be either fluorine, chlorine, iodine, bromine, or mixtures thereof. Of these, fluorine and, particularly, chlorine are preferred. The halogen may be added to the carrier material in any suitable manner either during preparation of the carrier material or before or after the addition of the other components.

For example, the halogen may be added at any stage of the preparation of the carrier material or to the calcined carrier material as an aqueous solution of a suitable decomposable halogen-containing compound such as hydrogen fluoride, hydrogen chloride, hydrogen bromide, ammonium chloride, etc. The halogen component or a portion thereof may be combined with the carrier material during the impregnation of the latter with the platinum-group component; for example, through the utilization of a mixture of chloroplatinic acid and hydrogen chloride. In another situation, the alumina hydrosol which is one of the hereinabove preferred methods to form the alumina carrier material may contain halogen and thus contribute at least a portion of the halogen component to the final composite. In a preferred embodiment, halogen is included in the air atmosphere utilized during the final calcination step to promote dispersion of the platinum group metal and modifier components. The halogen is combined with the carrier material to result in a final composite that contains from 0.1 to 1.0 mass % halogen, calculated on an elemental basis.

Regardless of the details of how the components of the catalyst are combined with the porous carrier material, the catalyst composite will be dried at a temperature of from 100° to 320°C for a period of from 2 to 24 or more hours. The dried composite is finally calcined at a temperature of from 400° to 600°C in an air atmosphere for a period of from 0.1 to 10 hours to convert the metallic compounds substantially to the oxide form. The chloride content of the catalyst may be adjusted by including a halogen or halogen-containing compound in the air atmosphere. The use of both chlorine and hydrogen chloride is particularly preferred.

The resultant calcined composite is subjected to a substantially water-free reduction step prior to its use in the conversion of hydrocarbons. This step is designed to insure a uniform and finely divided dispersion of the metallic components. Preferably, substantially pure and dry hydrogen (i.e., less than 20 vol. ppm H₂O) is used as the reducing agent in this step. The reducing agent contacts the catalyst at conditions, including a temperature of from 200° to 650°C and for a period of from 0.5 to 10 hours, effective to reduce substantially all of the platinum group metal component to the metallic state.

### EXAMPLES

The following examples will serve to illustrate certain specific embodiments of the present invention.

One catalyst each of the prior art and of the present invention were prepared as described hereinafter and evaluated for isomerization of cresols. Relative performance was assessed by comparing yield efficiency at a range of isomerization conversion levels.

### Example 1

Catalyst X represents a catalyst of the prior art. This catalyst consisted essentially of approximately 11 mass % hydrogen-form ZSM-5 zeolite with the remainder being alumina binder. The zeolite was added to an alumina sol solution, prepared by digesting metallic aluminum in hydrochloric acid, in an amount sufficient to yield a zeolite content in the finished catalyst of 11 mass %. A second solution of hexamethylenetetramine (HMT) was prepared and added to the zeolite/alumina sol mixture to give homogeneous admixture. This admixture was then dispersed as droplets into an oil bath maintained at 93°C. The droplets remained in the oil bath at 150°C until they set and formed hydrogel spheres. These spheres were removed from the oil bath, water washed with a 0.5% ammonia/water solution, air dried, and calcined at a temperature of 650°C.

### Example 2

Catalyst Y was prepared in accordance with the invention, and comprised approximately 11 mass % hydrogen-form ZSM-5 zeolite, 0.23 mass % platinum and 0.51 mass % lead, with the remainder being alumina binder. The lead/platinum atomic ratio thus was about 2.0:1. The spherical catalyst was prepared by the oil-drop method in the same manner as Catalyst A, with the lead added as Pb(NO₃)₂ by coimpregnation along with platinum as chloroplatinic acid in a solution with 4 mass % hydrochloric acid and 3.5 mass % nitric acid based on the support. The impregnated spheres were oxidized at 525°C, chloride-adjusted to 0.6 mass % Cl at 525°C and reduced in H₂ at 565°C.

### Example 3

A feedstock of pure m-cresol was dried and isomerized in a pilot-plant test to compare the performance of Catalysts X and Y. Pilot-plant operating conditions comprised a temperature range of 350°-450°C, pressure of 6078 kPa (60 atmospheres), and liquid hourly space velocity of 1.0 hr⁻¹. The temperature was varied within the indicated ranges to develop a relationship between the degree of isomerization and product selectivity for each of Catalysts X and Y, as discussed below and illustrated in the Figure.

Comparative selectivity for Catalysts X and Y is shown in the Figure. Results are plotted as mass % yield of (p-cresol + o-cresol), relative to the m-cresol feedstock, against the % of equilibrium (p-cresol + o-cresol) in total cresols in the isomerized product. The cresol equilibrium composition was taken to be 55% m-cresol, 23% p-cresol and 22% o-cresol. Yields of (p-cresol + o-cresol) corresponding to 90% and 100% selectivity are plotted as diagonal lines. The Figure shows that selectivity for Catalyst Y of the invention is 90% or more in all of the tests, while Catalyst X of the prior art demonstrates much lower selectivity.

## Claims

1. A process for the isomerization of a feedstock containing a non-equilibrium mixture of alkylphenols which comprises contacting hydrogen and such non-equilibrium mixture at isomerization conditions with a catalyst comprising a combination of a platinum group metal component and a modifier selected from the group consisting of lead, tin, germanium and bismuth with a carrier material containing a pentasil zeolite and an inorganic oxide binder.

2. The process of Claim 1 wherein the alkylphenols comprise cresols.

3. The process of Claim 2 or 3 wherein the isomerization conditions comprise a temperature in the range of 250°C to 500°C, a pressure of from 101.3 to 7948 kPa (1 to 75 atmospheres), and a liquid hourly space velocity of from 0.5 to 30 hr⁻¹.

4. The process of Claim 1, 2 or 3 wherein the isomerization conditions comprise a hydrogen-to-alkylphenol mole ratio of from about 0.5:1 to about 15:1.

5. The process of any of Claims 1 to 4 wherein the Group VIII metal component comprises platinum in an amount of 0.01 to 2 mass % of the catalyst and the atomic ratio of modifier to platinum group metal is 2 to 10.

6. The process of any one of Claims 1 to 5 wherein the pentasil zeolite comprises from about 1 to 20 mass % of the carrier material and wherein the pentasil zeolite is selected from the Group consisting of ZSM-5, ZSM-11, ZSM-12, ZSM-23, ZSM-35, and ZSM-38.
